(19) Europäisches Patentamt
European Patent Office
Office européen des brevets

(11) Veröffentlichungsnummer: **0 296 374 B1**

(12) # EUROPÄISCHE PATENTSCHRIFT

(45) Veröffentlichungstag der Patentschrift: **07.04.93**

(51) Int. Cl.$^5$: **C07K 9/00**, A61K 37/02, A61K 37/64, C07K 1/02

(21) Anmeldenummer: **88108485.9**

(22) Anmeldetag: **27.05.88**

(54) **Glycosylierte Aminosäuren und Peptide.**

(30) Priorität: **29.05.87 DE 3718181**

(43) Veröffentlichungstag der Anmeldung:
**28.12.88 Patentblatt 88/52**

(45) Bekanntmachung des Hinweises auf die Patenterteilung:
**07.04.93 Patentblatt 93/14**

(84) Benannte Vertragsstaaten:
**AT BE CH DE ES FR GB GR IT LI LU NL SE**

(56) Entgegenhaltungen:
**EP-A- 0 175 290**
**EP-A- 0 176 913**

**CHEMICAL ABSTRACTS, Band 107, Nr. 15, 12. Okfober 1987, Seite 762, Zusammenfassung Nr. 134669m, Columbus, Ohio, US; H. KESSLER et al.: "Synthesis of O-(alpha-glyco)peptides by the N-iodosuccinimide method", & ANGEW. CHEM. 1987, 99(9), 919-21**

(73) Patentinhaber: **HOECHST AKTIENGESELLSCHAFT**
**Postfach 80 03 20**
**W-6230 Frankfurt am Main 80(DE)**

(72) Erfinder: **Kolar, Cenek, Dr.**
**Deutschhausstrasse 20**
**W-3550 Marburg(DE)**
Erfinder: **Kessler, Horst, Prof. Dr.**
**Friedrich-Stoltze-Strasse 53**
**W-6231 Schwalbach am Taunus(DE)**
Erfinder: **Kottenhahn, Matthias**
**Wildaustrasse 1b**
**W-6450 Hanau(DE)**
Erfinder: **Kling, Andreas**
**Pfingstbrunnenstrasse 56**
**W-6231 Schwalbach am Taunus(DE)**

EP 0 296 374 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft glycosylierte Aminosäuren und Peptide sowie ein Verfahren zu ihrer Herstellung. Diese glycosylierten Aminosäuren und Peptide zeigen in wäßrigem Medium eine verbesserte Löslichkeit als die entsprechenden nicht glycosylierten Verbindungen, sind wertvolle Zwischenverbindungen bei der Herstellung biologisch aktiver Glycopeptide und hemmen außerdem die Glycosidasen von Glycoproteinen.

Bekannte Verfahren zur Herstellung von Glycopeptiden sind z.B. in B. Weinstein, Chemistry and Biochemistry of Amino Acids, Peptides and Proteins, Vol. 7 (1983) 35-112; R.R. Schmidt, Angew. Chem. 98, (1986) 213-236 (Angew. Chem. Int. Ed. Engl. 25 (1986) 212-235) oder H. Kunz, Angew. Chem. 99 (1987) 297-311 (Angew. Chem. Int. Ed. Engl. 26 (1987) 294-308) beschrieben.

Der Erfindung liegt die Aufgabe zugrunde, neue glycosylierte Aminosäuren und Peptide mit verbesserter Wirkung sowie ein neues Verfahren zu deren Herstellung zu finden.

Diese Aufgabe wird erfindungsgemäß gelöst durch die Verbindungen der allgemeinen Formel I

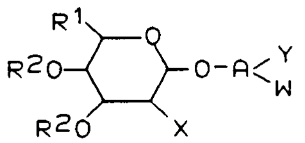

(I),

in welcher

- $R^1$ $CH_3$ oder $CH_2$-$OR^2$,
- $R^2$ unabhängig voneinander Wasserstoff, eine in der Kohlenhydratchemie übliche Schutzgruppe, ein Mono-, Di-oder Trisaccharid,
- A eine Hydroxyaminosäure,
- X Wasserstoff, Halogen, $N_3$, $NH_2$, NHAc, NH($C_1$-$C_6$)-Alkyl oder N(($C_1$-$C_6$)-Alkyl)$_2$,
- Y Wasserstoff, eine Urethanschutzgruppe oder eine oder mehrere gegebenenfalls geschützte Aminosäuren und
- W Hydroxy, eine Carboxylschutzgruppe oder eine oder mehrere gegebenenfalls geschützte Aminosäuren bedeuten, oder
- Y und W zusammen mit A für ein Cyclopeptid stehen,

wobei in den Verbindungen der Formel I 1-20 Aminosäuren vorliegen, mit der Maßgabe, daß falls Y und W zusammen mit A kein Cyclopeptid bilden, X für Wasserstoff oder Halogen steht.

Halogen kann Fluor, Chlor, Brom oder Iod sein; bevorzugt ist jedoch Brom oder Jod.
Alkyl kann geradkettig oder verzweigt vorliegen.

A steht für den Rest einer Hydroxyaminosäure, wie z.B. Ser, Thr, cis oder trans Hyp, 3Hyp, Tyr, Hyl, hSer, 2-Amino-5-hydroxy-valeriansäure oder 2-Amino-6-hydroxy-capronsäure. Y und W stehen u.a. für den Rest einer Aminosäure, vorzugsweise einer α-Aminosäure, die, falls chiral, in der D- oder L-Form vorliegen kann. D-Aminosäuren sind durch Voranstellen des Symbols "D" gekennzeichnet; Aminosäuren ohne Konfigurationssymbol sind L-konfiguriert. Bevorzugt sind natürlich vorkommende Aminosäuren (vgl. z.B. Wünsch et al., Synthese von Peptiden (Houben-Weyl 15/1 und 2), Stuttgart, Thieme 1974).

So kommen beispielsweise in Frage:
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hCys, His, hSer, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val.

$R^2$ steht u.a. für ein Mono-, Di- oder Trisaccharid wie beispielsweise die natürlichen, in Mikroorganismen, Pflanzen, Tieren oder Menschen vorkommenden D- oder L-Monosaccharide wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc) und N-Acetyl-mannosamin (ManNAc), Disaccharide, wie Maltose (Mal), Lactose (Lac), Cellobiose (Cel), Gentibiose (Gen), N-Acetyl-lactosamin (LacNAc), Chitobiose (Chit), β-Galactopyranosyl-(1→3)-N-acetyl-galactosamin und β-Galactopyranosyl-(1→3)- oder -(1→4)-N-acetyl-glucosamin oder für aus den vorstehend genannten Verbindungen gebildete Trisaccharide, sowie für deren synthetische Derivate, wie 2-Desoxy-, 2-Amino-, 2-Acetamido- oder 2-Halogeno-, bevorzugt Bromo- und Jodo-Zucker.

Die Auswahl der Schutzgruppen richtet sich nach den eingesetzten Zuckern und Aminosäuren bzw. Peptiden. Die Schutzgruppen werden in der Literatur üblichen Weise abgekürzt (vgl. z.B. Wünsch et al., Synthese von Peptiden (Houben-Weyl 15/1 und 2), Stuttgart, Thieme 1974).

Unter einer in der Kohlenhydratchemie üblichen Schutzgruppe versteht man z.B. die ($C_1$-$C_{11}$-Acylschutzgruppen wie ($C_1$-$C_6$)-Alkylcarbonyl (z.B. Acetyl, Trichloracetyl, Trifluoracetyl, Pivaloyl), ($C_1$-$C_6$)-Alkoxycarbonyl, ($C_7$-$C_{11}$)-Aralkylcarbonyl (z.B. Benzoyl, p-Nitrobenzoyl) oder Benzoyloxycarbonyl; ($C_1$-$C_4$)-Alkyl, ($C_6$-$C_{14}$)-Aryl-($C_1$-$C_4$)-alkyl (z.B. Benzyl) sowie gegebenenfalls modifizierte Methyl-, Methyloxymethyl-, Tetrahydropyranyl-, Benzyliden-, Isopropyliden- oder Trityl-Gruppen, wobei hier die Acylschutzgruppen, insbesondere die Acetyl-, Pivaloyl- und Benzoyl-Gruppe, sowie Benzyl, Benzyliden, Isopropyliden bevorzugt sind (vgl. auch E.E. Büllesbach, Kontakte (Merck) 1/80, 23-35; Schröder, Lübke, The Peptides Vol. 1, 1965).

Geeignete Urethanschutzgruppen und Carboxylschutzgruppen sind bei A. Hubbuch (Kontakte (Merck), 3/79, 14-23) und Schröder, Lübke, (The Peptides, Vol. 1, 1965) beschrieben. Bevorzugte Urethanschutzgruppen sind Fmoc, Aloc, Pyoc, Boc, Peoc, Dim, Dmoc, Z und DDZ. Bei den Carboxylschutzgruppen seien OBzl, OAl und OMe besonders genannt.

Die Verbindungen der Formel I können auch als deren physiologisch verträgliche Salze vorliegen.

Bevorzugt sind Verbindungen der allgemeinen Formel I, in welcher

| | |
|---|---|
| $R^1$ | $CH_2OR^2$, |
| $R^2$ | unabhängig voneinander ($C_1$-$C_4$)-Alkyl, ($C_6$-$C_{14}$)-Aryl-($C_1$-$C_4$)-alkyl, ($C_1$-$C_6$)-Alkylcarbonyl, ($C_7$-$C_{11}$)-Aralkylcarbonyl, insbesondere Acetyl, Pivaloyl, Benzoyl, Benzyl, Benzyliden, Isopropyliden oder ein Mono- oder Disaccharid aus der Gruppe Xyl, Glc, Man, Gal, Tal, Fuc, Rha, GlcNAc, GalNAc, ManNAc, Lac, Cel, LacNAc, β-Galactopyranosyl-(1→3)-N-acetylgalactosamin, β-Galactopyranosyl-(1→3)-N-acetyl-glucosamin, β-Galactopyranosyl-(1→4)-N-acetyl-glucosamin oder deren synthetische Derivate, |
| A | Ser, Thr, Hyp, 3Hyp, hSer, 2-Amino-5-hydroxy-valeriansäure oder 2-Amino-6-hydroxy-capronsäure, |
| X | Wasserstoff, Brom, Iod, $N_3$, $NH_2$ oder NHAc, |
| Y | Wasserstoff, Fmoc, Aloc, Pyoc, Boc, Peoc, Dim, Dmoc, Z, DDZ oder eine oder mehrere gegebenenfalls geschützte Aminosäuren aus der Gruppe Gly, Leu, Phe, Pro, Ala, Val, Glu, Ile, Tyr, Arg, Ser, Thr, Gln, Asp, Asn, Cys, Hyl, Hyp, Lys, Met, Orn, insbesondere Gly, Leu, Phe, Pro, Ala, Val, Glu, Ile, Thr, Lys, Gln und |
| W | Hydroxy, OAl, OMe, OBzl oder eine oder mehrere gegebenenfalls geschützte Aminosäuren aus der Gruppe Gly, Ala, Leu, Phe, Pro, Val, Glu, Ile, Tyr, Arg, Ser, Thr, Gln, Asp, Asn, Cys, Hyl, Hyp, Lys, Met, Orn, insbesondere Gly, Ala, Leu, Phe, Pro, Val, Glu, Ile, Thr, Lys, Gln, bedeuten oder |
| Y und W | zusammen mit A für ein Cyclopeptid stehen, mit der Maßgabe, daß falls Y und W zusammen mit A kein Cyclopeptid bilden, X für Wasserstoff oder Halogen steht. |

Die Verbindungen der Formel I enthalten vorzugsweise 1-10 Aminosäuren.

Die Erfindung betrifft ferner ein Verfahren zur Herstellung von Verbindungen der allgemeinen Formel I, das dadurch gekennzeichnet ist, daß man eine Aminosäure oder ein Peptid mit freier Hydroxygruppe, deren N- und C-terminale Gruppen geschützt sind, mit einem Glycal, d.h. einer ungesättigten 1,2-Glycopyranose, in Gegenwart von N-Brom- oder N-Iodsuccinimid in einem organischen Lösungsmittel umsetzt, die Glycokonjugate gegebenenfalls zu 2-Desoxy-glycosiden dehalogeniert, eine oder mehrere zum Schutz funktioneller Gruppen eingeführte Schutzgruppen abspaltet und die so erhaltenen Verbindungen gegebenenfalls in ihre Derivate überführt.

Die Kondensation nach dem erfindungsgemäßen Verfahren erfolgt bei einer Reaktionstemperatur zwischen 0 °C und 60 °C, vorzugsweise zwischen 25 °C und 50 °C.

Geeignete organische Lösungsmittel sind aprotische Lösungsmittel, besonders polare, aprotische Lösungsmittel wie z.B. Acetonitril, Chloroform, Methylenchlorid, Nitromethan, Tetrahydrofuran, Diglyme, Aceton.

Die Peptide können unter Benutzung der allgemeinen Methoden der Peptidchemie (Houben-Weyl, Methoden der Organischen Chemie, 15/1 und 2), beispielsweise stufenweise vom C-terminalen Ende oder durch Segment-Kondensation hergestellt werden. Cyclische Peptide werden aus ihren nach den obengenannten Methoden hergestellten linearen Vorläufern durch intramolekulare Kondensation des C- und N-terminalen Endes dargestellt, wie z.B. in J. Pharm. Sci., Vol.61, No.9 (1972) 1345-1356 beschrieben

Die nachstehenden Beispiele dienen zur Erläuterung der vorstehenden Erfindung, ohne daß sie darauf

beschränkt wäre.

**Beispiel 1**

Allgemeine Vorschrift:

a) Das entsprechende 3,4- oder 3,4,6-O- geschützte Glycal wird in absolutem $CH_3CN$ unter Schutzgas gelöst. Nach der Zugabe der C- und N-terminal geschützten Aminosäure bzw. des C- und N-terminal geschützten Peptides wird bei 0 °C unter Rühren und Schutzgas N-Brom- bzw. N-Iodsuccinimid (aus Tetrachlorkohlenstoff frisch umkristallisiert und getrocknet) zugegeben. Man läßt auf Raumtemperatur auftauen und rührt nach, bis die dünnschichtchromatographische Kontrolle kein Edukt mehr zeigt.
Man zieht das Lösungsmittel im Vakuum ab, nimmt in $CHCl_3$ auf, wäscht 2 mal mit 10 %iger Natriumthiosulfatlösung, 1 mal mit Eiswasser und trocknet die organische Phase über Magnesiumsulfat. Nach dem Einengen wird mit der entsprechenden Lösungsmittelkombination an Kieselgel chromatographiert. Das erhaltene Öl erstarrt im Ölpumpenvakuum zu festem, weißem Schaum.
b) Die aus Vorschrift a) erhaltenen Verbindungen werden in absolutem Benzol gelöst und unter Schutzgas und Rühren nacheinander mit katalytischen Mengen Azoisobutyronitril und 1,2 Äquivalenten Tributylzinnhydrid versetzt. Man rührt bei 30-40 °C unter Schutzgas nach, bis nach dünnschichtchromatographischer Kontrolle kein Edukt mehr vorhanden ist. Anschließend wird im Vakuum eingeengt und mit der geeigneten Lösungsmittelkombination an Kieselgel chromatographiert. Das erhaltene gelbliche Öl wird durch Ausschütteln mit Natriumthiosulfatlösung und Eiswasser von anhängenden Iodresten befreit. Nach dem Trocknen über Magnesiumsulfat wird eingeengt und im Ölpumpenvakuum zu festem, weißem Schaum getrocknet.
c) Die N-terminale Deblockierung der nach b) hergestellten Substanzen erfolgt nach den klassischen Methoden der Peptidchemie.
α) Fmoc-Abspaltung
100 mg der N-terminal geschützten Verbindung werden in 1 ml (über KOH getrocknetem) Morpholin bei Raumtemperatur gerührt bis nach dünnschichtchromatographischer Kontrolle kein Edukt mehr vorhanden ist. Das Morpholin wird anschließend im Ölpumpenvakuum durch mehrfaches Ausschleppen mit Toluol entfernt. Chromatographie mit der geeigneten Lösungsmittelkombination an Kieselgel ergibt das erwünschte Produkt mit freier Aminofunktion.
β) Z-Abspaltung
Die N-terminal geschützte Verbindung wird in absolutem Essigester gelöst. Die Lösung wird mit Stickstoff gespült und anschließend mit 400 mg/mmol•Substanz Katalysator (Pd 10 %ig auf C) versetzt. Im schwachen Wasserstoffstrom wird hydriert bis nach dünnschichtchromatographischer Kontrolle kein Edukt mehr vorhanden ist. Man filtriert vom Katalysator ab, wäscht mehrfach gründlich mit absolutem Methanol nach und engt im Vakuum ein. Nach dem Ausschleppen mit absolutem $CHCl_3$ erhält man einen festen weißen Schaum.
d) Die C-terminale Deblockierung der Verbindungen erfolgt nach den klassischen Methoden der Peptidchemie.
α) Bzl-Abspaltung
Siehe Z-Abspaltung unter c).

Die folgenden Verbindungen der Beispiele 2-39 wurden nach der unter 1 beschriebenen Vorschrift hergestellt.

**Beispiel 2**

Fmoc-((O)-3,4,6-Tri-O-acetyl-2-desoxy-2-iodo-α-D-mannopyranosyl)Ser-OBzl MW: 814,91
Ausbeute:2 eq Glucal: 98 %; Ausbeute:2 eq Fmoc-Ser-OBzl: 78,8 %
$[\alpha]_D^{20}$ = +9,54 (c = 1,09 in $CHCl_3$)
$R_f$ ($CHCl_3$/Aceton = 6,5/1) : 0,63
MS (FAB): 816 (M + $H^+$)
$^1$H-NMR (270 MHz, $CDCl_3$): 7,78; 7,63; 7,25-7,43; 5,87; 5,32; 5,34; 5,18; 5,07; 4,62; 4,53; 4,42; 4,30; 4,27; 4,15; 3,99; 3,95; 2,12; 2,08; 2,04 ppm.

**Beispiel 3**

Fmoc-((O)-3,4,6-Tri-O-acetyl-2-desoxy-2-iodo-α-D-talopyranosyl)Ser-OBzl MW: 814,91
Ausbeute:2 eq Galactal: 69,9 %; Ausbeute:1,5 eq Fmoc-Ser-OBzl: 59,6 %
Nach D.C. und HPLC einheitlich. Im $^1$H-NMR noch geringe Verunreinigungen.
$R_f$ (Hex/EE/Tol = 7/7/2) : 0,47
$[\alpha]_D^{20}$ = +33,23 (c = 0,99 in $CHCl_3$)

**Beispiel 4**

Aloc-((O)-3,4,6-Tri-O-acetyl-2-desoxy-2-iodo-α-D-mannopyranosyl)Ser-OBzl MW: 677,44
Ausbeute:1:1 eq : 35 %; Ausbeute:1,5 eq Aloc-Ser-OBzl: 35 %
$[\alpha]_D^{20}$ : +8,91 (c=1,01 in $CHCl_3$)
$R_f$ (Hex/EE/Tol = 7/7/2) : 0,39
MS (FAB): 678 (M + $H^+$)

**Beispiel 5**

Aloc-((O)-3,4,6-Tri-O-acetyl-2-desoxy-2-iodo-α-D-talopyranosyl)Ser-OBzl MW: 677,44
$R_f$ ($CHCl_3$/Aceton = 6,5/1) : 0,62

**Beispiel 6**

Fmoc-((O)-3,4,6-Tri-O-acetyl-2-desoxy-2-iodo-α-D-mannopyranosyl)Thr-OBzl MW: 828,94
Ausbeute:2 eq Glucal 72,6 %
$[\alpha]_D^{20}$ : +13,86 (c = 1,025 in $CHCl_3$)
$R_f$ (Hex/EE/Tol = 7/7/2) : 0,48

**Beispiel 7**

Fmoc-((O)-3,4,6-Tri-O-acetyl-2-desoxy-2-iodo-α-D-talopyranosyl)Thr-OBzl MW: 828.94
Ausbeute:2 eq Galactal: 77,7 %
Nach D.C. und HPLC einheitlich. Im $^1$H-NMR noch Spuren Verunreinigung.
$[\alpha]_D^{20}$ = +30,1 (c = 1,033 in $CHCl_3$)
$R_f$ (Hex/EE/Tol = 7/7/2): 0,48

**Beispiel 8**

Z-Pro-((O)-3,4,6-Tri-O-acetyl-2-desoxy-2-iodo-α-D-mannopyranosyl)Ser-Ala-OBzl MW: 895,7
Ausbeute:2 eq Glucal: quantitativ
$[\alpha]_D^{20}$ : -12,44 (c = 0,9 in $CHCl_3$)
$R_f$ (Tol/EE = 1/4) : 0,38
$^{13}$C-NMR : 168,6-172,1; ca. 152; 127,7-136,3; 101,82; 69,64; 68,86; 67,52; 67,41; 67,01; 62,03; 60,89; 52,67; 48,46; 47,18; 29,81; 29,34; 24,54; 20,8-21,4; 18,4 ppm.

**Beispiel 9**

Z-Pro-((O)-3,4,6-Tri-O-acety-2-desoxy-2-iodo-α-D-talopyranosyl)Ser-Ala-OBzl MW: 895,7
Ausbeute:2 eq Galactal: 78,8 %
$[\alpha]_D^{20}$ : +4,98 (c = 1,025 in $CHCl_3$)
$R_f$ (Tol/EE = 1/4) : 0.39
MS (FAB): 896 (M + $H^+$)

**Beispiel 10**

Pyoc-Pro-((O)-3,4,6-Tri-O-acetyl-2-desoxy-2-iodo-α-D-mannopyranosyl)Ser-Ala-OBzl MW: 910,7
Ausbeute: 30,7 %
Zersetzung bei Raumtemperatur schon nach kurzer Zeit.

**Beispiel 11**

Z-Pro-((O)-3,4,6-Tri-O-acetyl-2-desoxy-2-iodo-α-D-mannopyranosyl)Thr-Ala-OBzl MW: 909,74
Ausbeute:2 eq Glucal: 85,7 %
$[\alpha]_D^{20}$ : -4,73 (c = 2,05 in $CHCl_3$)
$R_f$ (Tol/EE = 1/1) : 0,11

**Beispiel 12**

Z-Pro-((O)-3,4,6-Tri-O-acetyl-2-desoxy-2-iodo-α-D-talopyranosyl)Thr-Ala-OBzl MW: 909,74
Ausbeute:2 eq Galactal: 85,1 %
$[\alpha]_D^{20}$ : +9,2 (c = 1 in $CHCl_3$)
$R_f$ (Tol/EE = 1/1) : 0,1
MS (FAB): 910 (M + $H^+$)

**Beispiele 13**

Boc-Gly-Phe-Leu-((O)-3,4,6-Tri-O-acetyl-2-desoxy-2-iodo-α-D-mannopyranosyl)Ser-Gly-OMe MW: 991,93
Ausbeute:2 eq Glucal: 49,2 %
$R_f$ ($CHCl_3$/Aceton = 1/1) : 0,48
MS (FAB): 992 (M + $H^+$)

**Beispiel 14**

Fmoc-((O)-3,4,6-Tri-O-acetyl-2-desoxy-α-D-glucopyranosyl)Ser-OBzl MW: 689,01
Ausbeute: 91 %
$[\alpha]_D^{20}$ : +43,35 (c = 0,985 in $CHCl_3$)
$R_f$ ($CH_2Cl_2$/EE = 6/1) : 0,5
MS (FAB): 690 (M + $H^+$)
$^{13}$C-NMR : 169,61-170,46; 155,85; 119,89-143,71; 97,88; 69,21; 68,90; 68,60; 68,33; 67,37; 67,25; 62,15; 54,43; 47,06; 34,63; 20,78; 20,56 ppm.

**Beispiel 15**

Fmoc-((O)-3,4,6-Tri-O-acetyl 2 desoxy-α-D-galactopyranosyl)Ser-OBzl MW: 689,01
Ausbeute: 84 %
$[\alpha]_D^{20}$ : +52,4 (c = 0,75 in $CHCl_3$)
$R_f$ (Hex/EE/Tol = 7/7/2) : 0,36

**Beispiel 16**

Aloc-((O)-3,4,6-Tri-O-acetyl-2-desoxy-α-D-glucopyranosyl)Ser-OBzl MW: 551,54
Ausbeute: 63,9 %
$[\alpha]_D^{20}$ : +52,5 (c = 0,6 in $CHCl_3$)
$R_f$ (Hex/EE/Tol = 7/7/2) : 0,29

**Beispiel 17**

Z-Pro-((O)-3,4,6-Tri-O-acetyl-2-desoxy-α-D-glucopyranosyl)Ser-Ala-OBzl MW: 769,8
Ausbeute: 81,9 %
$[\alpha]_D^{20}$ : +9,82 (c = 1,09 in $CHCl_3$)
$R_f$ (Tol/EE = 1/4) : 0,32

**Beispiel 18**

Z-Pro-((O)-3,4,6-Tri-O-acetyl-2-desoxy-α-D-galactopyranosyl)Ser-Ala-OBzl MW: 769,8
Ausbeute: 92 %

$[\alpha]_D^{20}$ : +24,57 (c = 1,05 in $CHCl_3$)
$R_f$ (Tol/EE = 1/4) : 0,31
MS (FAB): 770 (M + $H^+$)

**Beispiel 19**

Z-Pro-((O)-3,4,6-Tri-O-acetyl-2-desoxy-α-D-glucopyranosyl)Thr-Ala-oBzl MW: 783,84
Ausbeute: 85,7 %
$[\alpha]_D^{20}$ : +11,68 (c= 1,036 in $CHCl_3$)
$R_f$ (Tol/EE = 1/4) : 0,3
MS (FAB): 784 (M + $H^+$)
$^1$H-NMR : 7,25-7,40; 7,26; 7,12; 6,85; 5,28; 5,15; 5,11-5,26; 4,90; 4,56; 4,49; 4,40; 4,36; 4,26; 4,09; 4,04; 3,44-3,68; 2,18; 2,08; 2,04; 1,98; 1,92; 1,80; 1,44; 1,19; 1,08 ppm.

**Beispiel 20**

H-((O))-3,4,6-Tri-O-acetyl-2-desoxy-α-D-glucopyranosyl)Ser-OBzl MW: 466,77
Ausbeute: 73,9 %
$[\alpha]_D^{20}$ : +54,54 (c = 1,08 in $CHCl_3$)
$R_f$ ($CHCl_3$/Aceton = 1/1) : 0,32

**Beispiel 21**

H-((O)-3,4,6-Tri-O-acetyl-2-desoxy-α-D-galactopyranosyl)Ser-OBzl MW: 466,77

**Beispiel 22**

Fmoc-((O)-3,4,6-Tri-O-acetyl-2-desoxy-α-D-galactopyranosyl)Ser-OH MW: 598,89
Ausbeute: 57,8 % (Verluste im Filter)
$[\alpha]_D^{20}$ : +89,11 (c = 0,854 in $CHCl_3$)
$R_f$ ($CHCl_3$/MeOH = 10/1) : 0,18

**Beispiel 23**

H-Pro-((O)-3,4,6-Tri-O-acetyl-2-desoxy-α-D-glucopyranosyl)Ser-Ala-OH MW: 545,54
Ausbeute: 90,1 %
$R_f$ ($CHCl_3$/MeOH = 10/1) : 0,15

**Beispiel 24**

H-pro-((O)-3,4,6-Tri-O-acetyl-2-desoxy-α-D-glucopyranosyl)Thr-Ala-OH MW: 559,58
Ausbeute: 93,9 %
$R_f$ ($CHCl_3$/MeOH = 10/1) : 0,16

**Beispiel 25**

Fmoc-Pro-((O)-3,4,6-Tri-O-acetyl-2-desoxy-2-iodo-α-D-mannopyranosyl)Ser-Ala-OBzl MW: 982,88
Ausbeute:2 eq Glucal: 90 %
$[\alpha]_D^{20}$ : - 10,48 (c = 1,5 in $CHCl_3$)
$R_f$ (Tol/EE = 1/1) : 0,15
MS (FAB): 983 (M + $H^+$)

**Beispiel 26**

Fmoc-Pro-((O)-3,4,6-Tri-O-acetyl-2-desoxy-2-iodo-α-D-talopyranosyl)Ser-Ala-OBzl MW: 982,88
Ausbeute:2 eq Galactal: 90 %
$[\alpha]_D^{20}$ : + 1,77 (c = 1,2 in $CHCl_3$)

$$[\alpha]^{20}_{365}: + 4,19 \ (c = 1,2 \text{ in } CHCl_3)$$

$R_f$ (Tol/EE = 1/1) : 0,15
MS (FAB): 983 (M + $H^+$); 1005 (M + $Na^+$)

**Beispiel 27**

Z-Phe-D-Pro-Phe-Ala-((O)-3,4,6-Tri-O-acetyl-2-desoxy-2-iodo-α-D-mannopyranosyl)Ser-Phe-OBzl MW: 1336,28
Ausbeute:3 eq Glucal: 41,4 %
$[\alpha]^{20}_{D}$ : -1,07 (c = 0,37 in $CHCl_3$)

$$[\alpha]^{20}_{365}: -8,85 \ (c = 0,37 \text{ in } CHCl_3)$$

$R_f$ ($CHCl_3$/Ac = 4/1): 0,25
MS (FAB): 1337 (M + $H^+$)

**Beispiel 28**

Z-Phe-D-Pro-Phe-Ala-((O)-3,4,6-Tri-O-acetyl-2-desoxy-2-iodo-α-D-talopyranosyl)Ser-Phe-OBzl MW: 1336, 28
$[\alpha]^{20}_{D}$ : + 9,67 (c = 0,85 in $CHCl_3$)
$R_f$ ($CHCl_3$/Ac = 4/1): 0,26
MS (FAB): 1337 (M + $H^+$)

**Beispiel 29**

Cyclo(Phe-D-Pro-Phe-Ala-((O)-3,4,6-Tri-O-acetyl-2-desoxy-2-iodo-α-D-mannopyranosyl)Ser-Phe)- MW:1094,02
Ausbeute 3 eq Glucal: 67,5 %
$R_f$ ($CHCl_3$/Ac = 1/1): 0,31
MS (FAB): 1095 (M + $H^+$)

**Beispiel 30**

Cyclo(Phe-D-Pro-Phe-Ala-((O)-3,4,6-Tri-O-acetyl-2-desoxy-2-iodo-α-D-talopyranosyl)Ser-Phe) MW: 1094,02
Ausbeute:3 eq Galactal: 74 %
$|\alpha|^{20}_{D}$ : + 23,54 (c = 0,43 in $CHCl_3$)
$R_f$ ($CHCl_3$/Ac = 1/3): 0,41
MS (FAB): 1095 (M + $H^+$); 1117 (M + $Na^+$)

**Beispiel 31**

Fmoc-((O)-3,4,6-Tri-O-acetyl-2-desoxy-2-iodo-α-D-mannopyranosyl)Hyp-OBzl MW: 840,72
Ausbeute: 3 eq Glucal: 63,7 %
$[\alpha]^{20}_{D}$ : - 4,72 (c = 0,51 in $CHCl_3$)
$R_f$ (Hex/EE/Tol = 7/7/2): 0,35
MS (FAB): 841 (M + $H^+$)

**Beispiel 32**

Boc-((O)-3,4,6-Tri-O-acetyl-2-desoxy-α-iodo-α-D-talopyranosyl)Hyp-OBzl MW: 718,6
Ausbeute:4 eq Glucal: 46,3 %
$[\alpha]^{20}_{D}$ : + 3,79 (c = 1,26 in $CHCl_3$)

$R_f$ ($CH_2Cl_2$/EE: 6/1): 0,48
MS(FAB): 725 (M + $Li^+$)

**Beispiel 33**

Fmoc-((O)-2′,3′,4′,6′-Tetra-O-acetyl-D-galactopyranosyl-β-(1,4)-3,6-di-O-acetyl-2-desoxy-2-iodo-α-D-glucopyranosyl)Ser-OBzl MW: 1104,08
Ausbeute:3 eq Lactal: 51 %
$[\alpha]_D^{20}$ : + 18,8 (c = 1,29 in $CHCl_3$)
MS (FAB): 1104 (M + $H^+$)
$R_f$ (Hex/EE: 1/1) = 0,41

**Beispiel 34**

Fmoc-((O)-2′,3′,4′,6′-Tetra-O-acetyl-D-galactopyranosyl-β-(1,4)-3,6-di-O-acetyl-2-desoxy-2-iodo-α-D-glucopyranosyl)Thr-OBzl MW: 1118,11
Ausbeute:0,5 eq Lactal: 35 %
$[\alpha]_D^{20}$ : + 20,77 (c = 0,852 in $CHCl_3$)
$R_f$ ($CH_2Cl_2$/EE = 6/1): 0,25
MS (FAB): 1118 (M + $H^+$)

**Beispiel 35**

Z-Pro-((O)-2′,3′,4′,6′-Tetra-O-acetyl-D-galactopyranosyl-β-(1,4)-3,6-di-O-acetyl-2-desoxy-2-iodo-α-D-glucopyranosyl)Ser-Ala-OBzl MW: 1183,94
Ausbeute:1,5 eq Lactal: 61,5 %
$R_f$ (Tol/EE = 1/5): 0,4
MS (FAB): 1184 (M + $H^+$)

**Beispiel 36**

Z-Pro-((O)-2′,3′,4′,6′-Tetra-O-acetyl-D-galactopyranosyl-β-(l,4)-3,6-di-O-acetyl-2-desoxy-2-iodo-α-D-glycopyranosyl)Thr-Ala-OBzl MW: 1197,98
Ausbeute:1,5 eq Lactal: 53,7 %
$[\alpha]_D^{20}$ : + 10,88 (c = 1,286 in $CHCl_3$)
$R_f$ (Tol/EE = 1/5): 0,41
MS (FAB): 1198 (M + $H^+$); 1220 (M + $Na^+$)

**Beispiel 37**

Cyclo(Phe-D-Pro-Phe-Ala-((O)-2′,3′,4′,6′-Tetra-O-acetyl-D-galactopyranosyl-β-(1,4)-3,6-di-O-acetyl-2-desoxy-2-iodo-α-D-glycopyranosyl)Ser-Phe) MW: 1383,18
Ausbeute:3,5 eq Lactal: 62,5 %
$[\alpha]_D^{20}$ : + 13,25 (c = 0,75 in $CHCl_3$)
$R_f$ ($CHCl_3$/Ac = 1/1): 0,38
MS (FAB): 1383 (M + $H^+$)

**Beispiel 38**

Z-Phe-D-Pro-Phe-Ala-((O)-2′,3′,4′,6′-Tetra-O-acetyl-D-galactopyranosyl-β-(1,4)-3,6-di-O-acetyl-2-desoxy-2-iodo-α-D-glycopyranosyl)Ser-Phe-OBzl MW: 1625,45
Ausbeute:3,5 eq Lactal: 48,4 %
$R_f$ ($CHCl_3$/Ac = 4/1): 0,18
MS (FAB): 1625 (M + $H^+$)

**Beispiel 39**

Z-((O)-3,4,6-Tri-O-acetyl-2-desoxy-2-iodo-α-D-talopyranosyl) L-α-amino-6-hydroxycapronsäure-Phe-OMe MW: 839,74
$R_f$ (Hex/EE = 1/1): 0,32
MS (FAB): 840 (M + $H^+$)
$^{13}$C-NMR (250 MHz; $CDCl_3$): 169-172; 156; 135-137; 127-130; 102,52; 68,02; 67,02; 66,56; 65,34; 61,89; 54,65; 53,11; 52,33; 37,74; 32,29; 28,86; 21,98; 21,59; 20,96; 20,83; 20,66 ppm.

Die angegebenen $^1$H-NMR-Daten wurden an einem 270 MHz-Gerät in $CDCl_3$ (innerer Standard TMS), die $^{13}$C-NMR-Daten an einem 300 MHz-Gerät in $CDCl_3$ ermittelt falls nicht anders angegeben.

Die erfindungsgemäßen Verbindungen zeigen nach Abspaltung der in der Kohlenhydratchemie üblichen Schutzgruppen eine verbesserte Löslichkeit als die nicht glycosylierten Aminosäuren bzw. Peptide in wäßrigem Medium. Ferner sind die Verbindungen der Formel I wertvolle Zwischenverbindungen bei der Herstellung biologisch aktiver Glycopeptide. Sie hemmen außerdem die Glycosidasen von Glycoproteinen.

**Beispiel 40**

Inhibition der Glycosidasen mit synthetischen Glycopeptiden

a) Inhibition von α-Glucosidase
Die Untersuchung wurde nach der Methode von H. Halvorson (Methods in Enzymology, Edit.: E.F. Neufeld u. V. Ginsburg, Vol.8 (1966) 559-562, Academic press) durchgeführt. Die Methode basiert auf der Messung von freigesetzten p-Nitrophenol, daß durch enzymatische Spaltung von p-Nitrophenyl-α-D-glucopyranosid entsteht. Die Freisetzung von p-Nitrophenol wurde durch die 2-Desoxy-α-D-glucopyrano syl-peptide inhibiert.

Testdurchführung 0,1 ml α-Glucosidase (600 U, Fa. Boehringer Mannheim) wurde mit 2,9 ml Kaliumphosphat-Puffer ((M/15); pH 6,9) versetzt. Die Probe wurde ca. 30 Minuten bei 30 °C stehengelassen und anschließend wurden der Lösung 0,1 ml p-Nitrophenyl-α-D-glucopyranosid (0,01 M) unter Rühren zugegeben. Nach 5 Minuten Inkubationszeit wurde die Aktivitätsbestimmung (Meßstrahlung: Hg 405 nm; Schichtdicke: 1 cm; Inkubationsvolumen: 1,02 ml; Temperatur 25 °C; Messung gegen Leerwert) durchgeführt. Durch die enzymatische Hydrolyse wird das eingesetzte p-Nitrophenyl-α-D-glucopyranosid zu 70 % in p-Nitrophenol und D-Glucose gespalten. Die enzymatische Hydrolyse wird mittels der Glycopeptide, die den 2-Desoxy-α-D-glucopyranosyl-Baustein enthalten, inhibiert. Hierbei wurden bei gleicher Versuchsdurchführung wie oben angegeben neben p-Nitrophenyl-α-D-glucopyranosid die Glycopeptide (0,1-0,001 M) zugesetzt.
b) Inhibition von α-Galactosidasen
Die Inhibition der α-Galactosidase (Fa. Boehringer Mannheim) wurde mit den entsprechenden 2-Desoxy-α-D-galactopyranosyl-peptiden wie in Beispiel 40a) beschrieben durchgefürt.

**Beispiel 41**

Löslichkeit der erfindungsgemäßen Glycopeptide

Die Löslichkeit der Glycopeptide wurde in Wasser und wäßrigen Puffersystem (isotonische NaCl-Lösung) sowie Mannit-Lösung untersucht. Die erfindungsgemäßen Glycopeptide weisen hierbei wesentlich bessere Löslichkeit auf als die "Back bone"-Peptide.

**Verwendete Abkürzungen:**

| | |
|---|---|
| Ac | Acetyl |
| Aloc | Allyloxycarbonyl |
| Boc | tert.-Butyloxycarbonyl |
| D.C. | Dünnschichtchromatographie |
| DDZ | αα-Dimethyl-3,5-dimethoxy-benzyloxycarbonyl |
| Dim | 1,3-Dithian-2-yl-methylester |
| Dmoc | 1,3-Dithian-2-yl-methoxycarbonyl |
| EE | Essigsäureethylester |

| | |
|---|---|
| FAB | Fast atom bombardment |
| Fmoc | 9-Fluorenyl-methyl-oxycarbonyl- |
| Hex | n-Hexan |
| MeOH | Methanol |
| OAl | Allyl-ester |
| OBzl | Benzyl-ester |
| OMe | Methyl-ester |
| Peoc | 2-Phosphinoethoxycarbonyl |
| Pyoc | Pyridyl-(4)-methoxycarbonyl- |
| Tol | Toluol |
| Z | Benzyloxycarbonyl- |

**Patentansprüche**

**Patentansprüche für folgende Vertragsstaaten : AT, BE, CH, DE, FR, GB, IT, LI, LU, NL, SE**

**1.** Verbindung der allgemeinen Formel I

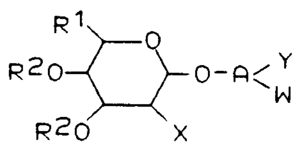

(I),

dadurch gekennzeichnet, daß

| | |
|---|---|
| $R^1$ | $CH_3$ oder $CH_2$-$OR^2$, |
| $R^2$ | unabhängig voneinander Wasserstoff, eine in der Kohlenhydratchemie übliche Schutzgruppe, ein Mono-, Di- oder Trisaccharid, |
| A | eine Hydroxyaminosäure, |
| X | Wasserstoff, Halogen, $N_3$, $NH_2$, NHAc, NH($C_1$-$C_6$)-Alkyl oder N(($C_1$-$C_6$)-Alkyl)$_2$, |
| Y | Wasserstoff, eine Urethanschutzgruppe oder eine oder mehrere gegebenenfalls geschützte Aminosäuren und |
| W | Hydroxy, eine Carboxylschutzgruppe oder eine oder mehrere gegebenenfalls geschützte Aminosäuren bedeuten, oder |
| Y und W | zusammen mit A für ein Cyclopeptid stehen, |

wobei in der Verbindung der Formel I 1-20 Aminosäuren vorliegen, mit der Maßgabe, daß falls Y und W zusammen mit A kein Cyclopeptid bilden, X für Wasserstoff oder Halogen steht.

**2.** Verbindung der allgemeinen Formel I, gemäß Anspruch 1, dadurch gekennzeichnet, daß

| | |
|---|---|
| $R^1$ | $CH_2OR^2$, |
| $R^2$ | unabhängig voneinander ($C_1$-$C_4$)-Alkyl, ($C_6$-$C_{14}$)-Aryl-($C_1$-$C_4$)-alkyl, ($C_1$-$C_6$)-Alkylcarbonyl, ($C_7$-$C_{11}$)-Aralkylcarbonyl, oder ein Mono- oder Disaccharid aus der Gruppe Xyl, Glc, Man, Gal, Tal, Fuc, Rha, GlcNAc, GalNAc, ManNAc, Lac, Cel, LacNAc, β-Galactopyranosyl-(1→3)-N-acetylgalactosamin, β-Galactopyranosyl-(1→3)-N-acetyl-glucosamin, β-Galactopyranosyl-(1→4)-N-acetyl-glucosamin oder deren synthetische Derivate, |
| A | Ser, Thr, Hyp, 3Hyp, hSer, 2-Amino-5-hydroxy-valeriansäure oder 2-Amino-6-hydroxycapronsäure, |
| X | Wasserstoff, Brom, Iod, $N_3$, $NH_2$ oder NHAc, |
| Y | Wasserstoff, Fmoc, Aloc, Pyoc, Boc, Peoc, Dim, Dmoc, Z, DDZ oder eine oder mehrere gegebenenfalls geschützte Aminosäuren aus der Gruppe Gly, Leu, Phe, Pro, Ala, Val, Glu, Ile, Tyr, Arg, Ser, Thr, Gln, Asp, Asn, Cys, Hyl, Hyp, Lys, Met, Orn, |
| W | Hydroxy, OAl, OMe, OBzl oder eine oder mehrere gegebenenfalls geschützte Aminosäuren aus der Gruppe Gly, Ala, Leu, Phe, Pro, Val, Glu, Ile, Tyr, Arg, Ser, Thr, Gln, Asp, Asn, Cys, Hyl, Hyp, Lys, Met, Orn bedeuten oder |
| Y und W | zusammen mit A für ein Cyclopeptid stehen, wobei in der Verbindung der Formel I 1-10 Aminosäuren vorliegen, mit der Maßgabe, daß falls Y und W zusammen mit A kein |

Cyclopeptid bilden, X für Wasserstoff oder Halogen steht.

3. Verfahren zur Herstellung einer Verbindung der allgemeinen Formel I gemäß einem oder mehreren der Ansprüche 1 bis 2, dadurch gekennzeichnet, daß man eine Aminosäure oder ein Peptid mit freier Hydroxygruppe, deren N- und C-terminale Gruppen geschützt sind, mit einem Glycal in Gegenwart von N-Brom- oder N-Jodsuccinimid in einem organischen Lösungsmittel umsetzt, die Glycokonjugate gegebenenfalls zu 2-Desoxy-Glycosiden dehalogeniert, eine oder mehrere zum Schutz funktioneller Gruppen eingeführte Schutzgruppen abspaltet und die so erhaltene Verbindung gegebenenfalls in ihre Derivate überführt.

4. Verwendung einer Verbindung gemäß einem oder mehreren der Ansprüche 1 bis 2, als Inhibitor der Glycosidasen von Glycoproteinen.

5. Pharmazeutisches Mittel enthaltend eine Verbindung gemäß einem oder mehreren der Ansprüche 1 bis 2.

**Patentansprüche für folgende Vertragsstaaten : ES, GR**

1. Verfahren zur Herstellung einer Verbindung der allgemeinen Formel I

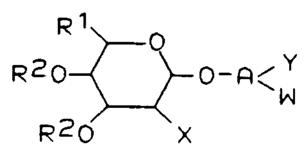

(I),

dadurch gekennzeichnet, daß

| | |
|---|---|
| $R^1$ | $CH_3$ oder $CH_2$-$OR^2$, |
| $R^2$ | unabhängig voneinander Wasserstoff, eine in der Kohlenhydratchemie übliche Schutzgruppe, ein Mono-, Di- oder Trisaccharid, |
| A | eine Hydroxyaminosäure, |
| X | Wasserstoff, Halogen, $N_3$, $NH_2$, NHAc, NH($C_1$-$C_6$)-Alkyl oder N(($C_1$-$C_6$)-Alkyl)$_2$, |
| Y | Wasserstoff, eine Urethanschutzgruppe oder eine oder mehrere gegebenenfalls geschützte Aminosäuren und |
| W | Hydroxy, eine Carboxylschutzgruppe oder eine oder mehrere gegebenenfalls geschützte Aminosäuren bedeuten, oder |
| Y und W | zusammen mit A für ein Cyclopeptid stehen, |

wobei in der Verbindung der Formel I 1-20 Aminosäuren vorliegen, mit der Maßgabe, daß falls Y und W zusammen mit A kein Cyclopeptid bilden, X für Wasserstoff oder Halogen steht, dadurch gekennzeichnet ist, daß man eine Aminosäure oder ein Peptid mit feier Hydroxygruppe, deren N- und C-terminale Gruppen geschützt sind, mit einem Glycal in Gegenwart von N-Brom- oder N-Iod-succinimid in einem organischen Lösungsmittel umsetzt, die Glycokonjugate gegebenenfalls zu 2-Desoxy-glycosiden dehalogeniert, eine oder mehrere zum Schutz funktioneller Gruppen eingeführte Schutzgruppen abspaltet und die so erhaltenen Verbindungen gegebenenfalls in ihre Derivate überführt.

2. Verfahren gemäß Anspruch 1, dadurch gekennzeichnet, daß man eine Verbindung der allgemeinen Formel I herstellt, in welcher

| | |
|---|---|
| $R^1$ | $CH_2OR^2$, |
| $R^2$ | unabhängig voneinander ($C_1$-$C_4$)-Alkyl, ($C_6$-$C_{14}$)-Aryl-($C_1$-$C_4$)-alkyl, ($C_1$-$C_6$)-Alkylcarbonyl, ($C_7$-$C_{11}$)-Aralkylcarbonyl, oder ein Mono- oder Disaccharid aus der Gruppe Xyl, Glc, Man, Gal, Tal, Fuc, Rha, GlcNAc, GalNAc, ManNAc, Lac, Cel, LacNAc, β-Galactopyranosyl-(1→3)-N-acetylgalactosamin, β-Galactopyranosyl-(1→3)-N-acetyl-glucosamin, β-Galactopyranosyl-(1→4)-N-acetyl-glucosamin oder deren synthetische Derivate, |
| A | Ser, Thr, Hyp, 3Hyp, hSer, 2-Amino-5-hydroxy-valeriansäure oder 2-Amino-6-hydroxy-capronsäure, |

| | |
|---|---|
| X | Wasserstoff, Brom, Iod, $N_3$, $NH_2$ oder NHAc, |
| Y | Wasserstoff, Fmoc, Aloc, Pyoc, Boc, Peoc, Dim, Dmoc, Z, DDZ oder eine oder mehrere gegebenenfalls geschützte Aminosäuren aus der Gruppe Gly, Leu, Phe, Pro, Ala, Val, Glu, Ile, Tyr, Arg, Ser, Thr, Gln, Asp, Asn, Cys, Hyl, Hyp, Lys, Met, Orn, |
| W | Hydroxy, OAl, OMe, OBzl oder eine oder mehrere gegebenenfalls geschützte Aminosäuren aus der Gruppe Gly, Ala, Leu, Phe, Pro, Val, Glu, Ile, Tyr, Arg, Ser, Thr, Gln, Asp, Asn, Cys, Hyl, Hyp, Lys, Met, Orn bedeuten oder |
| Y und W | zusammen mit A für ein Cyclopeptid stehen, wobei in der Verbindung der Formel I 1-10 Aminosäuren vorliegen, mit der Maßgabe, daß falls Y und W zusammen mit A kein Cyclopeptid bilden, X für Wasserstoff oder Halogen steht. |

**3.** Verfahren zur Herstellung eines pharmazeutischen Mittels, enthaltend eine Verbindung der allgemeinen Formel I gemäß einem oder mehreren der Ansprüche 1 bis 2, dadurch gekennzeichnet, daß man diese und einen Träger und gegebenenfalls weitere Zusatz- oder Hilfsstoffe in eine geeignete Darreichungsform bringt.

**Claims**

**Claims for the following Contracting States : AT, BE, CH, DE, FR, GB, IT, LI, LU, NL, SE**

**1.** A compound of the formula I

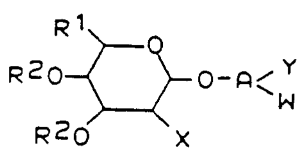

(I)

in which

| | |
|---|---|
| $R^1$ | denotes $CH_3$ or $CH_2$-$OR^2$, |
| $R^2$ | denote, independently of one another, hydrogen, a protective group customary in carbohydrate chemistry, or a mono-, di- or trisaccharide, |
| A | denotes a hydroxy amino acid, |
| X | denotes hydrogen, halogen, $N_3$, $NH_2$, NHAc, NH($C_1$-$C_6$)-alkyl or N(($C_1$-$C_6$)-alkyl)$_2$, |
| Y | denotes hydrogen, a urethane protective group or one or more optionally protected amino acids, and |
| W | denotes hydroxyl, a carboxyl protective group or one or more optionally protected amino acids, or |
| Y and W | together with A represent a cyclopeptide, |

with there being 1-20 amino acids in the compound of the formula I,

with the proviso that, if Y and W together with A do not form a cyclopeptide, X represents hydrogen or halogen.

**2.** A compound of the formula I as claimed in claim 1, in which

| | |
|---|---|
| $R^1$ | denotes $CH_2OR^2$, |
| $R^2$ | denote, independently of one another, ($C_1$-$C_4$)-alkyl, ($C_6$-$C_{14}$)-aryl-($C_1$-$C_4$)-alkyl, ($C_1$-$C_6$)-alkylcarbonyl, ($C_7$-$C_{11}$)-aralkylcarbonyl, or a mono- or disaccharide from the group comprising Xyl, Glc, Man, Gal, Tal, Fuc, Rha, GlcNAc, GalNAc, ManNAc, Lac, Cel, LacNAc, $\beta$-galactopyranosyl-(1→3)-N-acetylgalactosamine, $\beta$-galactopyranosyl-(1→3)-N-acetylglucosamine, $\beta$-galactopyranosyl-(1→4)-N-acetylglucosamine or the synthetic derivatives thereof, |
| A | denotes Ser, Thr, Hyp, 3Hyp, hSer, 2-amino-5-hydroxyvaleric acid or 2-amino-6-hydroxycaproic acid, |
| X | denotes hydrogen, bromine, iodine, $N_3$, $NH_2$ or NHAc, |
| Y | denotes hydrogen, Fmoc, Aloc, Pyoc, Boc, Peoc, Dim, Dmoc, Z, DDZ or one or more optionally protected amino acids from the group comprising Gly, Leu, Phe, Pro, Ala, |

Val, Glu, Ile, Tyr, Arg, Ser, Thr, Gln, Asp, Asn, Cys, Hyl, Hyp, Lys, Met and Orn, and

W denotes hydroxyl, OAl, OMe, OBzl or one or more optionally protected amino acids from the group comprising Gly, Ala, Leu, Phe, Pro, Val, Glu, Ile, Tyr, Arg, Ser, Thr, Gln, Asp, Asn, Cys, Hyl, Hyp, Lys, Met and Orn, or

Y and W together with A represent a cyclopeptide, with there being 1-10 amino acids in the compound of the formula I, and with the proviso that, if Y and W together with A do not form a cyclopeptide, X represents hydrogen or halogen.

**3.** A process for the preparation of a compound of the formula I as claimed in one or more of claims 1 and 2, which comprises reaction of an amino acid or of a peptide which has a free hydroxyl group and whose N- and C-terminal groups are protected with a glycal in the presence of N-bromo- or N-iodosuccinimide in an organic solvent, dehalogenation of the glycoconjugates, where appropriate, to 2-deoxyglycosides, elimination of one or more protective groups which have been introduced to protect functional groups, and, where appropriate, conversion of the resulting compound into its derivatives.

**4.** The use of a compound as claimed in one or more of claims 1 and 2 as an inhibitor of glycosidases of glycoproteins.

**5.** A pharmaceutical agent containing a compound as claimed in one or more of claims 1 and 2.

**Claims for the following Contracting States : ES, GR**

**1.** A process for the preparation of a compound of the formula I

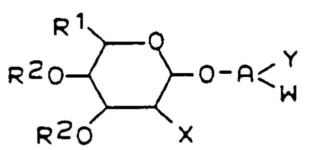

(I)

in which

R[1] denotes $CH_3$ or $CH_2$-$OR^2$,

R[2] denote, independently of one another, hydrogen, a protective group customary in carbohydrate chemistry, or a mono-, di- or trisaccharide,

A denotes a hydroxy amino acid,

X denotes hydrogen, halogen, $N_3$, $NH_2$, NHAc, NH($C_1$-$C_6$)-alkyl or N(($C_1$-$C_6$)-alkyl)$_2$,

Y denotes hydrogen, a urethane protective group or one or more optionally protected amino acids, and

W denotes hydroxyl, a carboxyl protective group or one or more optionally protected amino acids, or

Y and W together with A represent a cyclopeptide,

with there being 1-20 amino acids in the compound of the formula I,

with the proviso that, if Y and W together with A do not form a cyclopeptide, X represents hydrogen or halogen, which comprises reaction of an amino acid or of a peptide which has a free hydroxyl group and whose N- and C-terminal groups are protected with a glycal in the presence of N-bromo-or N-iodosuccinimide in an organic solvent, dehalogenation of the glycoconjugates, where appropriate, to 2-deoxyglycosides, elimination of one or more protective groups which have been introduced to protect functional groups, and, where appropriate, conversion of the resulting compounds into their derivatives.

**2.** A process as claimed in claim 1, wherein a compound of the formula I is prepared, in which

R[1] denotes $CH_2OR^2$,

R[2] denote, independently of one another, ($C_1$-$C_4$)-alkyl, ($C_6$-$C_{14}$)-aryl-($C_1$-$C_4$)-alkyl, ($C_1$-$C_6$)-alkylcarbonyl, ($C_7$-$C_{11}$)-aralkylcarbonyl, or a mono- or disaccharide from the group comprising Xyl, Glc, Man, Gal, Tal, Fuc, Rha, GlcNAc, GalNAc, ManNAc, Lac, Cel, LacNAc, β-galactopyranosyl-(1→3)-N-acetylgalactosamine, β-galactopyranosyl-(1→3)-N-acetylglucosamine, β-galactopyranosyl-(1→4)-N-acetylglucosamine or the synthetic de-

| | |
|---|---|
| | rivatives thereof, |
| A | denotes Ser, Thr, Hyp, 3Hyp, hSer, 2-amino-5-hydroxyvaleric acid or 2-amino-6-hydroxycaproic acid, |
| X | denotes hydrogen, bromine, iodine, $N_3$, $NH_2$ or NHAc, |
| Y | denotes hydrogen, Fmoc, Aloc, Pyoc, Boc, Peoc, Dim, Dmoc, Z, DDZ or one or more optionally protected amino acids from the group comprising Gly, Leu, Phe, Pro, Ala, Val, Glu, Ile, Tyr, Arg, Ser, Thr, Gln, Asp, Asn, Cys, Hyl, Hyp, Lys, Met and Orn, and |
| W | denotes hydroxyl, OAl, OMe, OBzl or one or more optionally protected amino acids from the group comprising Gly, Ala, Leu, Phe, Pro, Val, Glu, Ile, Tyr, Arg, Ser, Thr, Gln, Asp, Asn, Cys, Hyl, Hyp, Lys, Met and Orn, or |
| Y and W | together with A represent a cyclopeptide, with there being 1-10 amino acids in the compound of the formula I, and with the proviso that, if Y and W together with A do not form a cyclopeptide, X represents hydrogen or halogen. |

**3.** A process for the preparation of a pharmaceutical agent containing a compound of the formula I as claimed in one or more of claims 1 and 2, which comprises converting the latter and a vehicle and, where appropriate, further additives or auxiliaries into a suitable form for administration.

**Revendications**
**Revendications pour les Etats contractants suivants : AT, BE, CH, DE, FR, GB, IT, LI, LU, NL, SE**

**1.** Composé de formule générale I

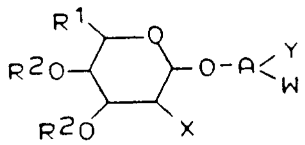

(I)

caractérisé en ce que

| | |
|---|---|
| $R^1$ | représente $CH_3$ ou $CH_2$-$OR^2$, |
| les radicaux $R^2$ | représentent, indépendamment l'un de l'autre, un atome d'hydrogène, un groupe protecteur usuel dans la chimie des hydrates de carbone, un mono-, di- ou trisaccharide, |
| A | représente un hydroxyaminoacide, |
| X | représente un atome d'hydrogène ou d'halogène ou $N_3$, ou un groupe $NH_2$, NHAc, NH-alkyle($C_1$-$C_6$) ou N-[alkyle($C_1$-$C_6$)]$_2$, |
| Y | représente un atome d'hydrogène ou un groupe protecteur en fonction uréthanne ou un ou plusieurs aminoacides éventuellement protégés, et |
| W | représente le groupe hydroxy, un groupe protecteur de fonction carboxy ou un ou plusieurs aminoacides éventuellement protégés, ou |
| Y et W, | conjointement avec A, représentent un cyclopeptide, |

1-20 aminoacides étant présents dans les composés de formule I,
étant entendu que lorsque Y et W, conjointement avec A, ne forment pas un cyclopeptide, X représente un atome d'hydrogène ou d'halogène.

**2.** Composé de formule générale I selon la revendication 1, caractérisé en ce que

| | |
|---|---|
| $R^1$ | représente $CH_2OR^2$, |
| les radicaux $R^2$ | représentent, indépendamment l'un de l'autre, un groupe alkyle en $C_1$-$C_4$, aryl-($C_6$-$C_{14}$)-alkyle($C_1$-$C_4$), alkyl($C_1$-$C_6$)-carbonyle, aralkyl($C_7$-$C_{11}$)-carbonyle ou un mono- ou disaccharide choisi parmi Xyl, Glc, Man, Gal, Tal, Fuc, Rha, GlcNAc, GalNAc, ManNAc, Lac, Cel, LacNAc, la $\beta$-galactopyrannosyl-(1→3)-N-acétylgalactosamine, la $\beta$-galactopyrannosyl-(1→3)-N-acétylglucosamine, la $\beta$-galactopyrannosyl-(1→4)-N-acétylglucosamine ou leurs dérivés synthétiques, |
| A | représente Ser, Thr, Hyp, 3Hyp, hSer, l'acide 2-amino-5-hydroxyvalérique ou l'acide 2-amino-6-hydroxycaproïque, |
| X | représente un atome d'hydrogène, de brome ou d'iode, $N_3$, $NH_2$ ou NHAc, |

| | |
|---|---|
| Y | représente un atome d'hydrogène, Fmoc, Aloc, Pyoc, Boc, Peoc, Dim, Dmoc, Z, DDZ ou un ou plusieurs aminoacides éventuellement protégés, choisi(s) parmi Gly, Leu, Phe, Pro, Ala, Val, Glu, Ile, Tyr, Arg, Ser, Thr, Gln, Asp, Asn, Cys, Hyl, Hyp, Lys, Met, Orn, |
| W | représente le groupe hydroxy, OAl, OMe, OBzl ou un ou plusieurs aminoacides éventuellement protégés, choisi(s) parmi Gly, Ala, Leu, Phe, Pro, Val, Glu, Ile, Tyr, Arg, Ser, Thr, Gln, Asp, Asn, Cys, Hyl, Hyp, Lys, Met, Orn, ou |
| Y et W | représentent, conjointement avec A, un cyclopeptide, 1-10 aminoacides étant présents dans le composé de formule I, étant entendu que lorsque Y et W, conjointement avec A, ne forment pas un cyclopeptide, X représente un atome d'hydrogène ou d'halogène. |

**3.** Procédé pour la préparation d'un composé de formule générale I selon une ou plusieurs des revendications 1 et 2, caractérisé en ce que l'on fait réagir un aminoacide ou un peptide à groupe hydroxy libre, dont les groupes N- et C- terminaux sont protégés, avec un glycal en présence de N-bromo- ou N-iodosuccinimide, dans un solvant organique, on soumet éventuellement les glycoconjugués à une déshalogénation en 2-désoxyglycosides, on élimine un ou plusieurs groupes protecteurs introduits pour la protection de groupes fonctionnels, et éventuellement on convertit en ses dérivés le composé ainsi obtenu.

**4.** Utilisation d'un composé selon une ou plusieurs des revendications 1 et 2, en tant qu'inhibiteur des glycosidases de glycoprotéines.

**5.** Produit pharmaceutique contenant un composé selon une ou plusieurs des revendications 1 et 2.

**Revendications pour les Etats contractants suivants : ES, GR**

**1.** Procédé pour la préparation d'un composé de formule générale I

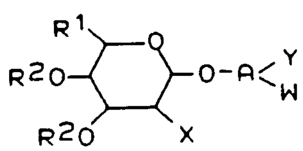

(I)

caractérisé en ce que

| | |
|---|---|
| $R^1$ | représente $CH_3$ ou $CH_2$-$OR^2$, |
| les radicaux $R^2$ | représentent, indépendamment l'un de l'autre, un atome d'hydrogène, un groupe protecteur usuel dans la chimie des hydrates de carbone, un mono-, di- ou trisaccharide, |
| A | représente un hydroxyaminoacide, |
| X | représente un atome d'hydrogène ou d'halogène ou $N_3$, ou un groupe $NH_2$, NHAc, NH-alkyle($C_1$-$C_6$) ou N-[alkyle($C_1$-$C_6$)]$_2$, |
| Y | représente un atome d'hydrogène ou un groupe protecteur en fonction uréthanne ou un ou plusieurs aminoacides éventuellement protégés, et |
| W | représente le groupe hydroxy, un groupe protecteur de fonction carboxy ou un ou plusieurs aminoacides éventuellement protégés, ou |
| Y et W, | conjointement avec A, représentent un cyclopeptide, |

1-20 aminoacides étant présents dans les composés de formule I,
étant entendu que lorsque Y et W, conjointement avec A, ne forment pas un cyclopeptide, X représente un atome d'hydrogène ou d'halogène, caractérisé en ce que l'on fait réagir un aminoacide ou un peptide à groupe hydroxy libre, dont les groupes N- et C- terminaux sont protégés, avec un glycal en présence de N-bromo- ou N-iodosuccinimide, dans un solvant organique, on soumet éventuellement les glycoconjugués à une déshalogénation en 2-désoxyglycosides, on élimine un ou plusieurs groupes protecteurs introduits pour la protection de groupes fonctionnels, et éventuellement on convertit en

leurs dérivés les composés ainsi obtenus.

**2.** Procédé selon la revendication 1, caractérisé en ce que l'on prépare un composé de formule générale I dans lequel

| | |
|---|---|
| $R^1$ | représente $CH_2OR^2$, |
| les radicaux $R^2$ | représentent, indépendamment l'un de l'autre, un groupe alkyle en $C_1$-$C_4$, aryl-($C_6$-$C_{14}$)-alkyle($C_1$-$C_4$), alkyl($C_1$-$C_6$)-carbonyle, aralkyl($C_7$-$C_{11}$)-carbonyle ou un mono- ou disaccharide choisi parmi Xyl, Glc, Man, Gal, Tal, Fuc, Rha, GlcNAc, GalNAc, ManNAc, Lac, Cel, LacNAc, la $\beta$-galactopyrannosyl-(1→3)-N-acétylgalactosamine, la $\beta$-galactopyrannosyl-(1→3)-N-acétylglucosamine, la $\beta$-galactopyrannosyl-(1→4)-N-acétylglucosamine ou leurs dérivés synthétiques, |
| A | représente Ser, Thr, Hyp, 3Hyp, hSer, l'acide 2-amino-5-hydroxyvalérique ou l'acide 2-amino-6-hydroxycaproïque, |
| X | représente un atome d'hydrogène, de brome ou d'iode, $N_3$, $NH_2$ ou NHAc, |
| Y | représente un atome d'hydrogène, Fmoc, Aloc, Pyoc, Boc, Peoc, Dim, Dmoc, Z, DDZ ou un ou plusieurs aminoacides éventuellement protégés, choisi(s) parmi Gly, Leu, Phe, Pro, Ala, Val, Glu, Ile, Tyr, Arg, Ser, Thr, Gln, Asp, Asn, Cys, Hyl, Hyp, Lys, Met, Orn, |
| W | représente le groupe hydroxy, OAl, OMe, OBzl ou un ou plusieurs aminoacides éventuellement protégés, choisi(s) parmi Gly, Ala, Leu, Phe, Pro, Val, Glu, Ile, Tyr, Arg, Ser, Thr, Gln, Asp, Asn, Cys, Hyl, Hyp, Lys, Met, Orn, ou |
| Y et W | représentent, conjointement avec A, un cyclopeptide, 1-10 aminoacides étant présents dans le composé de formule I, étant entendu que lorsque Y et W, conjointement avec A, ne forment pas un cyclopeptide, X représente un atome d'hydrogène ou d'halogène. |

**3.** Procédé pour la fabrication d'un produit pharmaceutique, contenant un composé de formule générale I, selon une ou plusieurs des revendications 1 et 2, caractérisé en ce qu'on met celui-ci sous une forme pharmaceutique appropriée, avec un véhicule et éventuellement d'autres adjuvants ou additifs.